# EUROPEAN PATENT APPLICATION

(11) **EP 0 528 080 A1**
(43) Date of publication of application: **24.02.1993**
(21) Application number: 91307323.5
(22) Date of filing: 09.08.1991
(51) Int. Cl.: A61F 2/30, A61F 2/38, A61L 27/00, A61B 17/58, A61L 31/00

(54) **Articular cartilage repair piece**

(71) Applicant: STRYKER CORPORATION, Kalamazoo, Michigan 49003-4085 (US)
(72) Inventor: Richmond,James W., Kalamazoo,Michigan (US); Block,John J., Rumson New Jersey 07760 (UA)
(74) Representative: Smith, Philip Antony

(57) **Abstract**

An articular cartilage repair piece (25) to replace a cut-out piece (24) of damaged (23) articular cartilage (22) on a bone (F) in an articulated joint (10) in a mammal includes a backing layer (30) of non-woven, felted fibrous material which is conformable to flat and curved surfaces. The front face (32) of the backing layer (30) is covered by a coating (33) of tough pliable material having front surfaces (35) which is tough, smooth and slippery in the presence of the natural synovial fluid of the joint (10) and responds naturally as the repair site interfaces with underlying meniscus cartilage (16,17) or articulair cartilage of an opposing surface through articulating motion in the joint (10) of a patient.

The repair piece (25) is temporarily fixed in place by resorbable pins (43) passing through the backing layer (30) and the coating (33).

## Description

This invention relates to an articular surface repair and more particularly to an articular surface implant.

The present invention was developed particularly in connection with repairing of injuries in the articular cartilage of a human knee joint. While the present invention is believed applicable to repair of similar injuries in the articular cartilage in other joints in humans and other mammals, for convenience same will be disclosed here in connection with repair of a defect in the articular cartilage covering a condyle of the femur in the knee joint of a human patient.

It has been known to employ a non-coated carbon fiber felt for articular surface repair. However, such is believed not to have proven entirely satisfactory.

Accordingly, the objects and purposes of this invention include provision of a method and apparatus for repair of a defect in the articular cartilage in a human or other mammal joint, for example on a condyle of the femur in a human knee joint, wherein usable materials include those used in other contexts in repairs in the human body and which have been approved by the FDA for other purposes within the human body, wherein such materials are commercially available, wherein such materials are of relatively low cost, wherein the completion of such a repair is within the skills of qualified orthopedic surgeons, and resorbable pins extend through holes through the repair piece and seat snugly in undersized holes in the underlying bone to temporarily fix the repair piece in place during healing and to allow movement of the joint earlier in the healing sequence.

Figure 1 is a fragmentary front elevational view of a human knee joint with sufficient tissue removed to show the articular cartilage on the condyles of the femur, and further showing a damaged area in such articular cartilage requiring repair.

Figure 2 is a side elevational view of the lower end of the femur of Figure 1 and is partially broken away, in correspondence with the sectional line II-II of Figure 1, to show in cross section a repair according to the present invention.

Figure 3 is an enlarged fraction of Figure 2 showing the repair piece (implant) in cross-section.

Figure 4 is an enlarged fragment of Figure 2 showing the repair implant initially affixed to the femur by elongate fasteners.

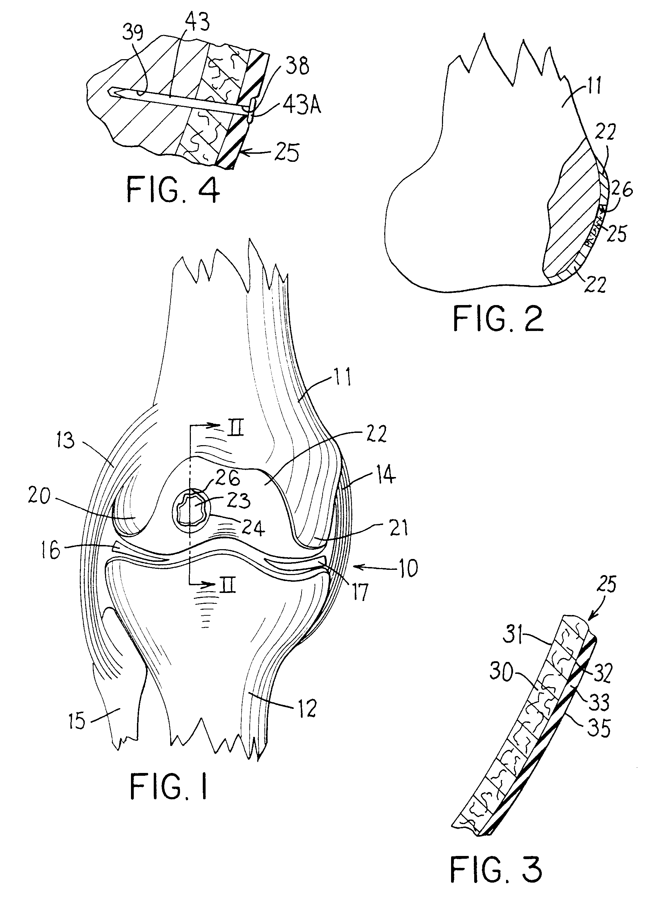

Although it is contemplated that the present invention is usable to repair defects in articular cartilage elsewhere in a human or other mammal body, for the sake of example, the invention is here illustrated in connection with repair of a defect in the articular cartilage on the femur in a human knee joint 10 illustrated in Figures 1 and 2.

Thus, Figure 1 illustrates a knee joint 10 between the bottom of a femur 11 and the top of a tibia 12. For clarity of illustration, only portions 13 and 14 of the connective tissue which movably ties the femur 11 to the underlying tibia 12 and fibula 15, is shown in Figure 1. Normally interposed between the opposing surfaces of the femur 11 and tibia 12 are lateral and medial meniscus cartilages 16 and 17. The convexly curved condyles 20 and 21 at the lower end of the femur 11 are normally supported by the meniscus cartilages 16 and 17, respectively, on the upper end of the tibia 12. Normally, the lower end of the femur 11, including the condyles 20 and 21, are covered by a layer 22 of cartilage material, referred to as the articular cartilage 22. The articular cartilage 22 forms a generally resilient padding which is fixed on the surface of the lower end of the femur 11 to protect the latter from wear and mechanical shock. Moreover, the articular cartilage 22, when lubricated by the fluid in the knee joint 10, provides a surface which is readily slidable on the underlying surfaces of the meniscus cartilages 16 and 17 (or on the upper surface of the tibia 12 should one or both of the meniscus cartilages 16 and 17 be partly or totally absent) during articulation of the knee joint 10.

A portion of the articular cartilage may become damaged (for example, be torn or holed) or become excessively worn. Figure 1 illustrates an example of a damaged area 23. Applicant has noted that it is desirable to repair such damage before it can spread or result in eventual injury or wear to the underlying condyle 20 or 21 or opposing tissues of the meniscus cartilage 16 or 17 or tissues associated with the facing portion of the tibia 12.

Generally, the present invention contemplates repairing the damaged area 23 by removing a conveniently shaped (here, for example, circular) portion 24 of the articular cartilage layer 22, including the damaged area 23. The size of the removed portion 24 is minimized, but it is large enough to include the entirety of the damaged area 23 therein. By making the removed portion 24 of regular shape (e.g., a circle, a square, etc.) it is easier to correctly shape and size a repair piece (implant) 25 hereafter described in more detail.

Following removal of the articular cartilage portion 24 including the damaged area 23, a correspondingly sized and shaped repair piece 25 (Figure 2) is inserted into the opening 26 left in the articular cartilage layer 22 upon removal of the portion 24 therefrom. The repair piece 25 is temporarily held in place by means hereinafter described, until such time as tissue in-growth, from the surface of the femur 11 and adjacent edges of the articular cartilage layer 22 surrounding the opening 26, more permanently fixes the repair piece 25 in place.

The similar size and shape (in plan) of the removed cartilage portion 24 and repair piece 25 can be achieved in alternative ways. The surgeon may remove only the damaged cartilage area and cut the repair piece to the shape of the latter. To assist the surgeon to cut the repair piece accurately to the shape of the removed damaged cartilage area, the surgeon can apply a maleable sheet of material, such as aluminum foil to the cut-out in the cartilage in such way that the maleable sheet material takes the outline of the cut-out and so can be used as a template to enable precise shaping and sizing of the repair piece. Alternately, as above discussed, the surgeon may remove a regularly shaped piece of cartilage including the damaged area and cut a repair piece to that shape.

The repair piece 25 (Figure 3) is cut to the desired shape and size from a previously prepared, wider panel (not shown). In the preferred embodiment shown, the repair piece (and the panel from which it is cut) is a substantially constant thickness laminate.

Such laminate comprises a non-woven, felted backing layer 30 (Figure 3) having a back face 31 intended to engage the surface of the condyle 20 where the portion 24 of the articular cartilage 22 has been removed. A backing layer thickness in the range of 1 to 10 mm is contemplated. In the preferred embodiment shown, the felted backing layer is of felted polytetrafluoroethylene (PTFE e.g. Teflon (TM)). The backing layer 30 has a front face 32. The laminate further comprises, in the preferred embodiment shown, a polyether urethane (hereinafter polyurethane or urethane) coating 33 covering and bonded to the front face 32 of the backing layer 30.

In one repair piece 25 constructed according to the invention, the felted Teflon (TM) backing 30 had a nominal thickness of about 1.2 mm and was obtained from C. R. Bard, Inc. located at Billerica, Massachusetts. Such felt has been used previously in ventricular aneurysmectomy, and tissue prosthesis, and suture buttressing, according to Bard.

It is also contemplated that a polyester (e.g. Dacron (TM)) felt material could be used for the backing 30. However, the Teflon (TM) felt material is preferred because it is a much more flexible material and results in a finished repair piece 25 that is more flexible and more conformable to curved or other irregular surfaces.

In the same unit according to the invention, the polyurethane coating 35 was obtained from Cardiac Control Systems, Inc. of Palm Coast, Florida, as a 20% solution of polyetherurethane and N,N-Dimethyacetamide (DMAC) under the trademark Surethane (TM). The recommended coating technique is by application of a series of multiple thin coats and curing each coat prior to application of the next coat. Curing of each polyurethane coat is by placement of the coated unit for a selected time in a controlled temperature and moisture chamber and thereafter boiling, or soaking in room temperature water, known techniques which need no further description. The curing of the polyurethane coating 33 solidifies the liquid into a pliable and conforming surface. The soaking or boiling process also eliminates substances, such as the solvent (DMAC), that otherwise may be toxic to the patient. By altering polymer concentration, application thickness, and/or curing duration between coats, a customized coating or film thickness can be achieved.

The polyurethane coating thickness at 33 is preferably in the range of about 1/2 mm to 2 mm, for example about 1 mm.

The polyurethane coating material penetrates the front face 32 of the felted backing 30 to a sufficient extent as to satisfactually mechanically interlock therewith, but the major thickness of the backing 30 is not penetrated by the polyurethane coating. The polyurethane coating thus does not substantially stiffen the backing 30, which is left in a flexible and conformable condition in the finished repair piece 25.

Applicant has been informed by the supplier, that Surethane has been used on FDA approved permanent pacemaker lead systems and permanent neural stimulation electrodes, and a physical testing has shown a final tensile strength of over 5500 PSI with a 700% to 800% maximum strain and has shown a soft-segment elasticity of approximately 500%. The Surethane coating 33 thus provides the repair piece 25 with a tough and durable front face.

The felted Teflon (TM) backing layer 30 is of a smooth limp material which is readily conformable to flat or curved surfaces. Thus, the Teflon (TM) felted backing 30 can easily conform to the curved shape of the condyle 20 at the location of the removed articular cartilage portion 24. Further, the back face 31 of the Teflon (TM) felted backing 30 readily receives tissue ingrowth from the opposed face of the femur 11 so that, in time, the repair piece 25 can become fixedly interlocked to the femur 11 by tissue ingrowth to the backface 31 and into the felted Teflon (TM) backing 30.

The polyurethane coating 33 on the front face of the backing 30 is flexible and does not significantly reduce the conformability of the Teflon (TM) backing 30. The polyurethane coating 33 does, however, provide a smooth, slippery and very tough front surface on the front face of the repair piece 25.

When lubricated by the lubricating liquid (synovial liquid) normally found within the joint, the front face 35 of the coating 33 provides a smooth and slippery sliding surface which simulates the front face of the natural undamaged articular cartilage 22 and is capable of sliding interaction with opposed tissue (for example, the lateral meniscus cartilage 16) and is readily slidable with respect thereto and rollable thereon without damaging such opposed tissue.

The shape and size, in plan, of the repair piece 25 is made to conform closely to that of the opening 26 so that the repair piece 25 fits snugly in edge-to-edge relation with the surrounding articular cartilage layer 22. To this end, the repair piece 25 is preferably cut from a larger panel by the surgeon at the time of surgery as above discussed. Also, the thickness of the repair piece 25 conforms generally to the depth of the defect. Thus, the repair piece 25 blends in as smoothly as possible with the surrounding cartilage 22 and will react with opposed tissue in the joint, as would have that portion 24 of the articular cartilage had it not been damaged and required replacement.

The repair piece 25 is preferably held in place at the repair site, until such time as it is anchored by normal tissue ingrowth from the underlying femur 11. In the preferred embodiment, the surgeon is supplied not only with a panel of the laminate from which the repair piece 25 is cut, but also with a punch or similar hole making device, as well as with resorbable headed pins, or tacks, 43 (Figure 4). At least one, and normally several, holes 38 are then punched, at the desired locations, through the layers 30 and 33 of the laminate, so as to extend through the entire thickness of the repair piece 25. With the repair piece 25 manually held in place in the opening 26 and against the bone 11, holes 39 of diameter somewhat less than that of the pins 43 are drilled in the surface of the bone 11 as coaxial continuations of the holes 38 in the repair piece 25.

Thereafter, with the repair piece 25 manually held in place in the opening 26 in the articular cartilage 22 and with the layer 30 thereof against the exposed surface of the bone 11, the pins 43 can be inserted through the respective holes 38 in the repair piece 25 and then can be urged into the aligned undersized holes 39 predrilled in the bone at 11. The diameter of the holes 39 in the bone is such that the pins 43 will go into place fairly easily. A thumb push will do oftentimes or light taps with a mallet may occasionally be required. The resorbable pins thus are not stressed or damaged during installation as might be the case if one attempted to pound them into the bone without predrilled holes.

The pins 43 have enlarged diameter heads 43A which are to retain the repair piece 25 in the opening 26. The heads 43A are preferably flat and are sufficiently inserted into the bone 11 as to abut the outer surface of the repair piece 25 and preferably be somewhat pressed thereinto.

Upon contact with body liquid in the joint, the pins 43 quickly swell and such swelling helps more firmly retain the pins 43A in their respective holes 39 in the bone 11. Thus, the swelling helps the pins to be more firmly self-retaining.

Since the repair piece 25 is fixedly held in place within the opening 26 and against the bone 11, joint rehabilitation can be started much earlier than would otherwise be the case and before significant, or at least substantial, tissue ingrowth into the repair piece 25 has taken place. For example, rather than having to prevent motion of the joint (as by retaining it in a cast) for six weeks or so, joint motion might be permitted in substantially lesser time, for example three weeks or even substantially less than that without risk of dislocating the repair piece 25 or otherwise damaging the repair. It is advantageous to be able to start rehabilitative joint movement early, for example to avoid adhesions between portions of the tissues in the joint which would normally be movable with respect to each other, to avoid muscle atrophy, etc., problems which may be especially severe in older people.

The resorbable pins 43 stay in position long enough for tissue ingrowth from the femur 11 into the felted backing 30 to occur to a sufficient extent as to anchor the repair piece 25A in place. Resorbable suture material used to form the pins 43 may for example be polyglycolic acid or polylactic acid or a composite of both, both being conventional materials for making bioresorbable sutures. The pins in one example were about 3/32 inch (about 2.5 mm) diameter by 1-1/2 inch (about 38 mm) long. The porous surface of the femur 11 under the articular cartilage 22 is soft enough to permit penetration by the exposed ends 45 of the pins.

While the pins 43 installed in this way are believed a particular suitable installation aid, it is also contemplated that other fasteners can be used.

It is contemplated that a combination of techniques may be used for temporary fixation of a repair piece 25 to the femur 11 prior to permanent fixation by tissue ingrowth in the manner above described. For example, bone cement may be added between the repair piece and bone in Figure 4 to further ensure fixed anchoring of the repair piece.

## Claims

1. An articular cartilage repair piece to be substituted for a removed piece of damaged articular cartilage on a bone in an articulated joint in a mammal, comprising:
a repair piece including a backing layer of non-woven, felted fibrous material, the backing layer being limp and readily conformable to flat and curved surfaces, the backing layer having a back face for application to the site of the damaged articular cartilage for eventual anchoring to the bone, the repair piece having a front face for opposing and coacting interface with adjacent material of the joint, said front face is defined by a coating of tough pliable material having a front surface which is smooth and is slippery in the presence of the natural synovial fluid in the joint;
means for temporarily fixing the repair piece in place of the cut-out piece of damaged articular cartilage, said temporary fixing means comprising resorbable fasteners extending at least through said backing layer and pliable material coating, the fasteners extending through and protruding beyond said back face of said backing layer for insertion into the bone exposed by removal of the cut-out piece.

2. The apparatus of Claim 1 in which said coating is of polyurethane.

3. The apparatus of Claim 2 in which the polyurethane coat is 1/2 to 2 mm thick.

4. The apparatus of Claim 1 in which the backing layer material is PTFE or polyester.

5. The apparatus of Claim 1 in which the backing layer material is PTFE.

6. The apparatus of Claim 4 or 5 in which said backing layer is from about 1 mm to 10 mm thick.

7. The apparatus of Claim 1 in which said temporary fixing means includes a bioadhesive layer applied as a short time fixative to said back face of said backing layer of said repair piece for application to and adhesion to the bone exposed by removal of the removed piece, wherein the bioadhesive layer may include fibrinogen and thrombin.

8. The apparatus of Claim 1 in which the fasteners are pins with flat enlarged diameter heads retaining the pliable material coating.

9. The apparatus of Claim 8 in which said fasteners consist of a material selected from the group consisting of polyglycolic acid or polylactic acid and a composite of polyglycolic and polylactic acids, the pins being approximately 2.5 mm in diameter and 38 mm long.

10. A method of repairing a damaged area of an articular cartilage covering the bone of an articulated joint in a mammal, comprising the steps of:
removing a piece of the articular cartilage, which piece includes the damaged area, so as to expose the underlying bone;
cutting a comparably sized and shaped repair piece from a piece of laminate comprising a backing layer of non-woven, felted fibrous material comprising PTFE or polyester, the backing layer having a front face defined by a coating of tough pliable material having a front surface which is smooth and is slippery in the presence of natural synovial fluid in the joint, the coating being of polyurethane, the backing layer having a back face;
making holes through the repair piece at desired locations thereon and of a size to snugly but easily slidably receive therethrough resorbable fasteners;
forming holes in the exposed portion of the underlying bone coaxially aligned with the holes in the repair piece and of diameter to frictionally receive the ends of the resorbable fasteners;
inserting the repair piece into the opening in the articular cartilage left by the cut-out piece which has been removed, with the back face of the repair piece facing the bone;
temporarily fixing the repair piece in place of the cut-out piece by inserting such resorbable fasteners into the holes in the repair piece and then pressing such fasteners into the holes in the exposed surface of the bone, to a depth that heads of the fasteners bear on the coating of the repair piece, so that the fasteners fix the repair piece in place of the cut-out piece at least until such time as tissue ingrowth from the bone into the back face of the repair piece holds the latter in place.
